# EUROPEAN PATENT APPLICATION

(11) **EP 4 487 835 A1**
(43) Date of publication of application: **08.01.2025**
(21) Application number: 23306164.7
(22) Date of filing: 07.07.2023
(51) Int. Cl.: A61K 8/9789, A61Q 19/08

(54) **USE OF WHOLE PLANTS IN COSMETIC COMPOSITIONS**

(71) Applicant: Shiseido Co., Ltd., Tokyo 104-0061 (JP)
(72) Inventor: AZPITARTE, Lindsay, 78150 LE CHESNAY (FR); ANDRE, Patrice, 45170 NEUVILLE AUX BOIS (FR); BURTIN, Frédéric, 69004 LYON (FR); ROCHET, Leïla, 75017 PARIS (FR); MOREAU, Deborah, 45000 ORLEANS (FR); JOUANNEAU, Emilie, 45000 ORLEANS (FR)
(74) Representative: Vial, Lionel

(57) **Abstract**

The present invention relates to a composition comprising, or consisting of, at least one liquid phase of a grind of a whole fresh plant.

## Description

### Field of the invention

The present invention relates to whole, fresh and preferably young plants for use in compositions, in particular cosmetic compositions. More particularly, the present invention relates to compositions comprising whole, fresh and young plants, their use in cosmetic compositions as well as processes for preparing the same.

### Background of the invention

The cosmetic industry is constantly evolving, with new challenges arising every day. Consumers are increasingly aware of the impact of the products they use on their health and the environment, and are looking for natural and sustainable alternatives. Thus, more and more cosmetic industries are looking to reduce their environmental footprint, to continue to propose innovative and effective products and to respond to consumer concerns about the need for greater transparency in the production and sourcing of cosmetic products.

There are many possible solutions to these challenges, but the use of plants in cosmetics is a promising option. Plants offer a source of natural, sustainable and renewable ingredients that can be used in a wide variety of cosmetic products. Plants contain natural compounds that have beneficial properties for the skin, such as antioxidants, vitamins and minerals. In addition, the use of plants in cosmetic products can help reduce the environmental impact of the cosmetics industry (Ribeiro et al. (2015) Cosmetics, 2: 48-65).

However, there are also drawbacks to using botanicals in cosmetics, particularly in terms of formulation. Plants can be difficult to work with and may require more complex technologies and formulation processes, some plants can be susceptible to oxidation and contamination, which can affect the quality and stability of the final product. In addition, problems related to the variability of the composition may occur because plants can vary considerably in terms of chemical composition depending on factors such as season, geography, growing conditions, etc.

Plant extracts are generally used in dry form, which is the usual way to preserve them before proceeding to the extraction of the so-called active molecules. However, the use of dry extracts or concentrates of plant parts does not allow to obtain the benefits associated with all the active ingredients of the plants as drying plant affects the composition and molecular biodiversity of them.

Thus, it is still necessary to provide cosmetically active ingredients with a great biomolecular diversity meeting the expectations of consumers in terms of safety, efficiency and environmental impact.

### Summary of the invention

The present invention arises from the unexpected finding by the inventors that a composition comprising at least one liquid phase of a grind of a whole fresh plant selected from the group consisting of *Centella asiatica, Coleus forskollii, Ocimum tenuiflorum* and mixture thereof, which were grown using indoor culture methods, presents a great molecular diversity and includes molecules called "discrete" which are not usually found in dry extracts of these same plants. The inventors have also evaluated the biological activity of this composition and discovered that it can be used as a cosmetic composition. In addition, the inventors have highlighted the complementary effects obtained by combining two or three of these plants.

The inventors have developed indoor cultivation techniques to produce the plants. These techniques allow to use whole, fresh, and young plants. This process also allows to cultivate plants in partially, or totally controlled conditions, ensures a great traceability of the plants and may also preferably ensure a standardization of the culture conditions contributing to the safety and the reproducibility of the composition. The inventors have also developed processing technique including a cryogenic grinding step of the fresh plant in its entirety in order to produce compositions comprising all the molecular diversity of the plant.

Thus, the present invention relates to a composition comprising, or consisting of, at least one liquid phase of a grind of a whole fresh plant.

The present invention also relates to a process for the preparation of a composition comprising, or consisting of, at least one liquid phase of a grind of a whole fresh plant comprising a step of cryogenic grinding of the whole fresh plant.

The present invention also relates to a composition intended to be used in cosmetic compositions prepared or capable of being prepared according to the process as defined above.

The present invention also relates to a cosmetic composition comprising the composition as defined above or the composition obtained by the process as defined.

The present invention also relates to a cosmetic composition, in particular a topical cosmetic composition, for use for preventing, reducing, ameliorating, improving, alleviating, and/or reducing effects of skin aging.

The present invention also relates to a method for preventing, reducing, ameliorating, improving, alleviating, and/or reducing effects of skin aging in an individual comprising applying to the individual a composition as defined above.

### Detailed description of the invention

In the specification and in the claims, the terms "including", "comprising" and "containing" can be used interchangeably. These terms are open-ended terms and should be interpreted to mean "including", but not limited to. Thus, when an object "comprises" or "contains" one or several elements, other elements than those mentioned may also be included in the object. These terms encompass the more restrictive terms "consisting essentially of" and "consisting of." When an object is said to "consist of" one or several elements, the object is limited to the listed elements and cannot include other elements than those mentioned.

As used herein, the terms "x% (w/w)" and "x% w/w" are equivalent to "x g per 100 g". Unless indicated otherwise, all % value shall be taken to indicate x% w/w.

In the context of this application, the term "at least" also includes the starting point of the open range. For example, an amount of "at least 1 % w/w" means any amount equal to 1 percentage by weight or above.

In the context of this application, the term "at most" also includes the ending point of the open range. For example, an amount of "at most 50 % w/w" means any amount equal to 50 percentage by weight or less.

### Whole fresh young plant

Preferably, the plant according to the invention is whole, fresh and young.

As intended herein, the expression "whole plant" refers to the plant in its entirety. Whole plants are well known by the person skilled in the art and may comprise all of its parts, preferably intact, including for example but not necessarily the root, the seed, the fruit, the stem, the leaf, the fruit, and the flower.

As intended herein the expression "fresh plant" means a plant that has not undergone any drying process of the plant or of a part of it. It refers to a plant that still contain its natural water content.

As intended herein the expression "young plant" depends on the species of the plant and the context in which it is cultivated. Typically a young plant according to the invention means a plant which is still in the growth phase and is preferably a few weeks old such as at least one week, 2 weeks, 3 weeks or 4 weeks old.

Preferably, the plant according to the invention is grown in indoor growing systems. Indoor growing is well known to the person skilled in the art and refers to the practice of cultivating plant inside an enclosed space using artificial light and other environmental controls. Indoor growing of plants allows for greater control over the growing condition. It typically involves the use of grow lights, which mimic the natural spectrum of sunlight and provide the energy that plants need for photosynthesis. Other environmental controls, such as temperature, humidity, and air circulation, are also carefully monitored and adjusted to ensure optimal growing conditions.

Indoor growing methods typically include vertical farming technologies, hydroponics culture, aeroponics culture, and aquaponics culture.

The person skilled in the art is able to choose the type of indoor culture suitable for the culture of the plant according to the invention. The choice of which method to use will depend on factors such as the type of plants being grown, available space, and desired level of control over the growing environment.

Vertical farming is the practice of growing where plants are grown in vertically stacked layers. It often incorporates Controlled Environment Agriculture(CEA), which aims to optimize plant growth. Vertical farming systems can be soil-based, hydroponic, or aeroponic, and can be configured in a variety of ways such as tower systems, wall systems and room systems. In tower system, plants are grown in stacked towers, with each layer receiving its own supply of water and nutrients. In wall systems, plants are grown on vertical walls, with each plant receiving its own supply of water and nutrients. In room systems, plants are grown in a fully enclosed room or building, with multiple levels of shelves or racks that can be adjusted to optimize plant growth and yield.

Hydroponics culture involves growing of plants with or without soil. The plant may for example grow with their roots exposed to water-based mineral nutrient solutions. In hydroponic systems, plants are typically grown in containers that are filled with an inert medium, such as rock wool, perlite, gravel, sand, wood fibers, clay pebbles, etc., containers may also be filled with soil, and are watered with a nutrient-rich solution. Hydroponic systems can range from simple, passive systems to complex, automated setups. Some common types of hydroponic systems include: deep water culture (DWC), dip irrigation, nutrient film technique (NFT), Ebb and flow (flood and drain) sub-irrigation, etc.

Aeroponics is a method of growing plants without soil or any other growing medium, by suspending the plant roots in a mist of nutrient-rich solution. In aeroponic systems, plants are typically grown in a chamber or container that is enclosed and has nozzles that spray a fine mist of nutrient solution onto the roots. The roots are continuously or discontinuously kept in an environment saturated with fine drops of mist or aerosol of nutrient solution, providing them with the water and nutrients they need to grow. The mist is usually applied for a few seconds every few minutes, ensuring that the roots are constantly moistened without being over-saturated.

Aquaponics culture combines hydroponics with aquaculture (fish farming), creating a symbiotic system where fish waste provides the nutrients that plants need to grow.

More preferably, the plant according to the invention is grown in aeroponics culture, even more preferably in vertical aeroponics culture.

Advantageously, the indoor culture allows for organic and local agriculture reducing the need for road or rail transport, it strongly or totally limit the need for insecticides, pesticides, herbicides and allows a controlled use of fertilizers such as chemical or organic fertilizers, it also allows for a better control of the environment of the plants, reducing their stress, offering to the plants what they need when they need it, reducing water consumption. Indoor culture is also a way to reduce health risk since it is practiced in a highly controlled indoor environment.

Preferably, the plant according to the invention is produced without the use of insecticides, pesticides, herbicides.

The plant according to the invention is preferably selected from the group consisting of *Centella asiatica, Coleus forskollii, Ocimum tenuiflorum* and mixtures thereof.

*Centella asiatica* is well known to the person skilled in the art. This plant is also known as Gotu kola, Kodavan, Indian pennywort and Asiatic pennywort. It is a herbaceous plant native to Asia, commonly used in traditional medicine.

*Coleus forskollii* is well known to the person skilled in the art. This plant is also known by the synonym Plectranthus barbatus. It is a tropical perennial plant that belongs to the mint family. This plant is native to India, Nepal, and Thailand and is commonly used in Ayurvedic medicine.

*Ocimum tenuiflorum* is well known to the person skilled in the art. This plant is also known as holy basil, Tulsi or Tulasi. It is an aromatic plant that belongs to the mint family. It is native to India and Southeast Asia, and is widely used in Ayurvedic medicine.

Advantageously, the plants *Centella asiatica, Coleus forskollii,* and *Ocimum tenuiflorum* produced according to the invention present a great complementarity when at least two of them are used together, it means that they have properties and synergic actions which reinforce each other within the composition according to the invention.

### Composition

### a. Liquid phase of a grind of a whole fresh plant

Preferably, the liquid phase of a grind according to the invention is obtained by grinding a plant according to the invention, preferably a whole, fresh, young plant as defined above.

Grinding is well known to the person skilled in the art. It typically involves the steps of selecting a plant, optionally cutting the plant into small pieces, pouring the plant pieces into a grinder and grind the plant. The time required depends on the type of grinder and can easily be adapted by the person skilled in the art. Then, the ground material can be filtrated in order to obtain a liquid phase of a grind and a solid phase of a grind.

Preferably, the liquid phase of a grind according to the invention is obtained by cryogenic grinding.

The grinding according to the invention preferably provides a liquid phase and a solid phase. Preferably, the liquid phase of a grind according to the invention comprises plant water and hydrophilic molecules. As intended herein, the expression "plant water" refers to the water made by the plant itself, in particular via the photosynthesis reaction.

Preferably, the composition according to the invention comprises the liquid phases of grinds of at least two different whole fresh plants according to the invention. Preferably, the composition according to the invention comprises the liquid phases of grinds of at least three different whole fresh plants according to the invention.

Preferably, the composition according to the invention comprises at least 1% w/w of the at least one liquid phase of a grind of a whole fresh plant according to the invention.

Preferably, the composition according to the invention comprises at most 30% w/w of the at least one liquid phase of a grind of a whole fresh plant according to the invention.

The composition according to the invention preferably comprises from 1 to 30% w/w, more preferably from 1 to 25% w/w of the at least one liquid phase of a grind of a whole fresh plant, such as from 3 to 25% w/w, from 5 to 25% w/w, from 10 to 25% w/w, from 15 to 25% w/w, from 20 to 25% w/w or from 1 to 20% w/w, from 1 to 15% w/w from 3 to 10% w/w, from 3 to 5% w/w of the at least one liquid phase of a grind of a whole fresh plant according to the invention.

In embodiments of the invention in which the composition comprises the liquid phase of grinds of two different whole fresh plants, the composition preferably comprises 10% w/w of the liquid phase of a grind of a whole fresh plant according to the invention and 90% w/w of the liquid phase of a grind of another whole fresh plant according to the invention, the plants being different and being selected from the group consisting of *Centella asiatica, Coleus forskollii,* and *Ocimum tenuiflorum,* or 20% w/w of the liquid phase of a grind of a whole fresh plant and 80% w/w of the liquid phase of a grind of anotherwhole fresh plant, or 30% w/w of the liquid phase of a grind of a whole fresh plant and 70% w/w of the liquid phase of a grind of another whole fresh plant, or 40% w/w of the liquid phase of a grind of a whole fresh plant and 60% w/w of the liquid phase of a grind of another whole fresh plant, or 50% w/w of the liquid phase of a grind of a whole fresh plant and 50% w/w of the liquid phase of a grind of another whole fresh plant, the plants being different and being selected from the group consisting of *Centella asiatica, Coleus forskollii,* and *Ocimum tenuiflorum.*

In embodiments of the invention in which the composition comprises the liquid phase of grinds of three different whole fresh plants according to the invention, the composition preferably comprises:
25% w/w *Coleus forskollii,* 25% w/w *Centella asiatica,* 25% w/w *Coleus forskollii*; or
27% w/w *Coleus forskollii,* 27% w/w *Centella asiatica,* 27% w/w *Centella asiatica;* or
30% w/w *Coleus forskollii,* 30% w/w *Centella asiatica,* 30% w/w *Ocimum tenuiflorum;* or
33% w/w *Coleus forskollii,* 33% w/w *Centella asiatica,* 33% w/w *Ocimum tenuiflorum;* or
10% w/w *Coleus forskollii,* 40% w/w *Centella asiatica,* 50% w/w *Ocimum tenuiflorum;* or
10% w/w *Coleus forskollii,* 50% w/w *Centella asiatica,* 40% w/w *Ocimum tenuiflorum;* or
40% w/w *Coleus forskollii,* 10% w/w *Centella asiatica,* 50% w/w *Ocimum tenuiflorum;* or
40% w/w *Coleus forskollii,* 50% w/w *Centella asiatica,* 10% w/w *Ocimum tenuiflorum;* or
50% w/w *Coleus forskollii,* 40% w/w *Centella asiatica,* 10% w/w *Ocimum tenuiflorum;* or
50% w/w *Coleus forskollii,* 10% w/w *Centella asiatica,* 40% w/w *Ocimum tenuiflorum;* or
30% w/w *Coleus forskollii,* 60% w/w *Centella asiatica,* 10% w/w *Ocimum tenuiflorum;* or
30% w/w *Coleus forskollii,* 10% w/w *Centella asiatica,* 60% w/w *Ocimum tenuiflorum;* or
60% w/w *Coleus forskollii,* 30% w/w *Centella asiatica,* 10% w/w *Ocimum tenuiflorum;* or
60% w/w *Coleus forskollii,* 10% w/w *Centella asiatica,* 30% w/w *Ocimum tenuiflorum;* or
10% w/w *Coleus forskollii,* 60% w/w *Centella asiatica,* 30% w/w *Ocimum tenuiflorum;* or
10% w/w *Coleus forskollii,* 30% w/w *Centella asiatica,* 60% w/w *Ocimum tenuiflorum;* or
60% w/w *Coleus forskollii,* 20% w/w *Centella asiatica,* 20% w/w *Ocimum tenuiflorum;* or
20% w/w *Coleus forskollii,* 60% w/w *Centella asiatica,* 20% w/w *Ocimum tenuiflorum;* or
20% w/w *Coleus forskollii,* 20% w/w *Centella asiatica,* 60% w/w *Ocimum tenuiflorum;* or
30% w/w *Coleus forskollii,* 30% w/w *Centella asiatica,* 40% w/w *Ocimum tenuiflorum;* or
40% w/w *Coleus forskollii,* 30% w/w *Centella asiatica,* 30% w/w *Ocimum tenuiflorum;* or
30% w/w *Coleus forskollii,* 40% w/w *Centella asiatica,* 30% w/w *Ocimum tenuiflorum;* or
50% w/w *Coleus forskollii,* 30% w/w *Centella asiatica,* 20% w/w *Ocimum tenuiflorum;* or
50% w/w *Coleus forskollii,* 20% w/w *Centella asiatica,* 30% w/w *Ocimum tenuiflorum;* or
30% w/w *Coleus forskollii,* 50% w/w *Centella asiatica,* 20% w/w *Ocimum tenuiflorum;* or
30% w/w *Coleus forskollii,* 20% w/w *Centella asiatica,* 50% w/w *Ocimum tenuiflorum;* or
20% w/w *Coleus forskollii,* 50% w/w *Centella asiatica,* 30% w/w *Ocimum tenuiflorum;* or
20% w/w *Coleus forskollii,* 30% w/w *Centella asiatica,* 50% w/w *Ocimum tenuiflorum;* or
20% w/w *Coleus forskollii,* 40% w/w *Centella asiatica,* 40% w/w *Ocimum tenuiflorum;* or
40% w/w *Coleus forskollii,* 20% w/w *Centella asiatica,* 40% w/w *Ocimum tenuiflorum;* or
40% w/w *Coleus forskollii,* 40% w/w *Centella asiatica,* 20% w/w *Ocimum tenuiflorum:* or
70% w/w *Coleus forskollii,* 10% w/w *Centella asiatica,* 20% w/w *Ocimum tenuiflorum;* or
70% w/w *Coleus forskollii,* 20% w/w *Centella asiatica,* 10% w/w *Ocimum tenuiflorum;* or
20% w/w *Coleus forskollii,* 70% w/w *Centella asiatica,* 10% w/w *Ocimum tenuiflorum;* or
20% w/w *Coleus forskollii,* 10% w/w *Centella asiatica,* 70% w/w *Ocimum tenuiflorum;* or
10% w/w *Coleus forskollii,* 70% w/w *Centella asiatica,* 20% w/w *Ocimum tenuiflorum;* or
10% w/w *Coleus forskollii,* 20% w/w *Centella asiatica,* 70% w/w *Ocimum tenuiflorum.*

More preferably, in embodiments of the invention in which the composition comprises the liquid phase of grinds of three different whole fresh plants, the composition preferably comprises 33% w/w *Coleus forskollii,* 33% w/w *Centella asiatica,* and 33% w/w *Ocimum tenuiflorum.*

The composition according to the invention preferably comprises at least one compound belonging to at least one phytochemical family selected from the group consisting of organic acids, nitrogenous compounds, phenolic compounds, sulphate compounds, osidic derivatives, lipids, terpene and mixture thereof. As intended herein, "phytochemical family" refers to groups of chemical compounds found in plants that have similar chemical structures.

### b. Liquid phase of an alcoholic macerate of a solid phase of a whole fresh plant

The composition according to the invention may further comprise at least one liquid phase of an alcoholic macerate of a solid phase of a whole fresh plant according to the invention. Preferably, the plant is as defined above. Preferably, the solid phase of a whole fresh plant according to the invention is a solid phase of a grind of at least one whole, fresh and young plant according to the invention.

Preferably, the solid phase of a grind of at least one whole, fresh and young plant is obtained by cryogenic grinding. In embodiments of the invention, the composition according to the invention comprises the liquid phase of a grind of a whole fresh plant and the liquid phase of an alcoholic macerate of a solid phase of the grind of the same whole fresh plant, both the liquid phase of a grind and the solid phase of a grind result from the same grinding process of the same plant, more preferably a cryogenic grinding process. Thus, in embodiments of the invention the composition comprises all the molecular diversity of at least one whole fresh plant according to the invention. In embodiments of the invention, at least one whole fresh plant according to the invention is used in its entirety in the composition.

Maceration is a process well known to the person skilled in the art. Typically, the plant is introduced in a clean dry jar or container and is completely recovered with a liquid, such as water, alcohol or oil. The liquid is preferably cold. The plant can be cut into small pieces before maceration. The plant is let in the liquid for several hours. Then, the plant is removed, for example by filtration, to obtain a liquid phase of a macerate.

Preferably, the ratio of the solid phase of a whole fresh plant that is macerated to the liquid according to the invention is comprised between 1:2 (1 part of the solid phase of a whole fresh plant that is macerated to 2 parts of liquid) to 1: 3.

Preferably, the solid phase of a whole fresh plant that is macerated is left in contact with the liquid for a time comprised between 5 and 25 hours, such as from 5 to 20 hours, from 5 to 15 hours, or from 5 to 10 hours.

Preferably, the liquid according to the invention is a water/alcohol mixture, more preferably a 50/50 alcohol/water mixture. Alcohol can be chosen among any alcohol suitable for the maceration of a plant. Preferably, the alcohol according to the invention is selected from the group consisting of methanol, ethanol, propanol, isopropanol, and butanol. More preferably, the alcohol according to the invention is ethanol.

Preferably, the composition according to the invention comprises the liquid phase of an alcoholic macerate of a solid phase of at least two different whole fresh plants according to the invention. Preferably, the composition according to the invention comprises the liquid phase of an alcoholic macerate of a solid phase of three different whole fresh plants according to the invention.

The plant according to the invention is preferably selected from the group consisting of *Centella asiatica, Coleus forskollii, Ocimum tenuiflorum* and mixtures thereof as defined above.

Preferably, the composition according to the invention comprises at least 3% w/w of the at least one liquid phase of an alcoholic macerate of a solid phase of at least one whole fresh plant, in particular a solid phase of a grind of at least one whole, fresh plant according to the invention.

Preferably, the composition according to the invention comprises at most 30% w/w of the at least one liquid phase of an alcoholic macerate of a solid phase of a whole fresh plant, in particular a solid phase of a grind of at least one whole, fresh plant according to the invention.

Preferably, the composition according to the invention preferably comprises from 3 to 30% w/w, more preferably from 3 to 25% w/w, such as from 5 to 25% w/w, from 10 to 25% w/w, from 15 to 25% w/w, from 20 to 25% w/w or from 3 to 20% w/w, from 3 to15% w/w from 3 to 10% w/w, from 3 to 5% w/w of the at least one liquid phase of an alcoholic macerate of a solid phase of a whole fresh plant, in particular a solid phase of a grind of at least one whole, fresh plant according to the invention.

In embodiments of the invention in which the composition comprises the liquid phase of an alcoholic macerate of a solid phase of at least two different whole fresh plants, the composition preferably comprises 10% w/w of the liquid phase of an alcoholic macerate of a solid phase of a whole fresh plant and 90% w/w of the liquid phase of an alcoholic macerate of a solid phase of another whole fresh plant, the plants being different and being selected from the group consisting of *Centella asiatica, Coleus forskollii,* and *Ocimum tenuiflorum,* or 20% w/w of the liquid phase of an alcoholic macerate of a solid phase of a whole fresh plant and 80% w/w of the liquid phase of an alcoholic macerate of a solid phase of another whole fresh plant, or 30% w/w of the liquid phase of an alcoholic macerate of a solid phase of a whole fresh plant and 70% w/w of the liquid phase of an alcoholic macerate of a solid phase of another whole fresh plant, or 40% w/w of the liquid phase of an alcoholic macerate of a solid phase of a whole fresh plant and 60% w/w of the liquid phase of an alcoholic macerate of a solid phase of another whole fresh plant, or 50% w/w of the liquid phase of an alcoholic macerate of a solid phase of a whole fresh plant and 50% w/w of the liquid phase of an alcoholic macerate of a solid phase of another whole fresh plant, the plants being different and being selected from the group consisting of *Centella asiatica, Coleus forskollii,* and *Ocimum tenuiflorum.*

In embodiments of the invention in which the composition comprises the liquid phase of an alcoholic macerate of a solid phase of three different whole fresh plants, the composition preferably comprises:
27% w/w *Coleus forskollii,* 27% w/w *Centella asiatica,* 27% w/w *Ocimum tenuiflorum;* or
30% w/w *Coleus forskollii,* 30% w/w *Centella asiatica,* 30% w/w *Ocimum tenuiflorum;* or
33% w/w *Coleus forskollii,* 33% w/w *Centella asiatica,* 33% w/w *Ocimum tenuiflorum;* or
10% w/w *Coleus forskollii,* 40% w/w *Centella asiatica,* 50% w/w *Ocimum tenuiflorum;* or
10% w/w *Coleus forskollii,* 50% w/w *Centella asiatica,* 40% w/w *Ocimum tenuiflorum;* or
40% w/w *Coleus forskollii,* 10% w/w *Centella asiatica,* 50% w/w *Ocimum tenuiflorum;* or
40% w/w *Coleus forskollii,* 50% w/w *Centella asiatica,* 10% w/w *Ocimum tenuiflorum;* or
50% w/w *Coleus forskollii,* 40% w/w *Centella asiatica,* 10% w/w *Ocimum tenuiflorum;* or
50% w/w *Coleus forskollii,* 10% w/w *Centella asiatica,* 40% w/w *Ocimum tenuiflorum;* or
30% w/w *Coleus forskollii,* 60% w/w *Centella asiatica,* 10% w/w *Ocimum tenuiflorum;* or
30% w/w *Coleus forskollii,* 10% w/w *Centella asiatica,* 60% w/w *Ocimum tenuiflorum;* or
60% w/w *Coleus forskollii,* 30% w/w *Centella asiatica,* 10% w/w *Ocimum tenuiflorum;* or
60% w/w *Coleus forskollii,* 10% w/w *Centella asiatica,* 30% w/w *Ocimum tenuiflorum;* or
10% w/w *Coleus forskollii,* 60% w/w *Centella asiatica,* 30% w/w *Ocimum tenuiflorum;* or
10% w/w *Coleus forskollii,* 30% w/w *Centella asiatica,* 60% w/w *Ocimum tenuiflorum;* or
60% w/w *Coleus forskollii,* 20% w/w *Centella asiatica,* 20% w/w *Ocimum tenuiflorum;* or
20% w/w *Coleus forskollii,* 60% w/w *Centella asiatica,* 20% w/w *Ocimum tenuiflorum;* or
20% w/w *Coleus forskollii,* 20% w/w *Centella asiatica,* 60% w/w *Ocimum tenuiflorum;* or
30% w/w *Coleus forskollii,* 30% w/w *Centella asiatica,* 40% w/w *Ocimum tenuiflorum;* or
40% w/w *Coleus forskollii,* 30% w/w *Centella asiatica,* 30% w/w *Ocimum tenuiflorum;* or
30% w/w *Coleus forskollii,* 40% w/w *Centella asiatica,* 30% w/w *Ocimum tenuiflorum;* or
50% w/w *Coleus forskollii,* 30% w/w *Centella asiatica,* 20% w/w *Ocimum tenuiflorum;* or
50% w/w *Coleus forskollii,* 20% w/w *Centella asiatica,* 30% w/w *Ocimum tenuiflorum;* or
30% w/w *Coleus forskollii,* 50% w/w *Centella asiatica,* 20% w/w *Ocimum tenuiflorum;* or
30% w/w *Coleus forskollii,* 20% w/w *Centella asiatica,* 50% w/w *Ocimum tenuiflorum;* or
20% w/w *Coleus forskollii,* 50% w/w *Centella asiatica,* 30% w/w *Ocimum tenuiflorum;* or
20% w/w *Coleus forskollii,* 30% w/w *Centella asiatica,* 50% w/w *Ocimum tenuiflorum;* or
20% w/w *Coleus forskollii,* 40% w/w *Centella asiatica,* 40% w/w *Ocimum tenuiflorum;* or
40% w/w *Coleus forskollii,* 20% w/w *Centella asiatica,* 40% w/w *Ocimum tenuiflorum;* or
40% w/w *Coleus forskollii,* 40% w/w *Centella asiatica,* 20% w/w *Ocimum tenuiflorum:* or
70% w/w *Coleus forskollii,* 10% w/w *Centella asiatica,* 20% w/w *Ocimum tenuiflorum;* or
70% w/w *Coleus forskollii,* 20% w/w *Centella asiatica,* 10% w/w *Ocimum tenuiflorum;* or
20% w/w *Coleus forskollii,* 70% w/w *Centella asiatica,* 10% w/w *Ocimum tenuiflorum;* or
20% w/w *Coleus forskollii,* 10% w/w *Centella asiatica,* 70% w/w *Ocimum tenuiflorum;* or
10% w/w *Coleus forskollii,* 70% w/w *Centella asiatica,* 20% w/w *Ocimum tenuiflorum;* or
10% w/w *Coleus forskollii,* 20% w/w *Centella asiatica,* 70% w/w *Ocimum tenuiflorum.*

### c. Additives

The composition according to the invention may further comprise at least one additional compound selected from the group consisting of formulation agents or additives with known, conventional use in cosmetic and dermatological compositions such as, as nonlimiting examples, emollients, colorants, film-forming agents, surfactants, perfumes, preservatives, emulsifiers, oils, glycols, vitamins such as vitamin E, UV filters, antimicrobial agents, antiseptic agents, preservative agents, antioxidant agents, and mixtures thereof.

Examples of such additives include caprylic/capric triglyceride glycerin, squalane, iron oxides, betaine, cetearyl alcohol, decyl glucoside, propanediol, citric acid, vinegar, 1,2-hexandiol, phenethyl alcohol, alpha glucan oligosaccharide, diglycerin, sodium benzoate, potassium sorbate, caprylyl glycol, cetearyl glucoside, glyceryl stearate, polyglyceryl 4 caprate stearic acid, PEG-40 stearatejojoba oil, xanthan gum, butylene glycol, vitamin C, vitamin E, beta carotènezinc oxide, panthenol, etc.

The person skilled in the art will know which formulation agents to add to the compositions according to the invention and in what amounts depending on the required properties.

### Process

### a) Cryogenic grinding

The step of cryogenic grinding according to the invention is preferably performed on at least one whole, fresh and young plant according to the invention and as defined above.

Cryogenic grinding is well known to the person skilled in the art. It is a type of grinding involving the cooling of the material to very low temperature using liquid nitrogen or other cryogenic gases. Typically, the material to be ground is first cooled to a temperature below its glass transition temperature, which is the temperature at which it becomes brittle and can be easily fractured, this can be done by placing the material in a freezer or by using a cryogenic cooling system. The material is then fed into a grinding mill that is cooled with liquid nitrogen or other cryogenic gases, which keeps the material at a very low temperature during the grinding process. The grinding mill can be a vibratory mill, a hammer mill, or an attrition mill. Advantageously, cryogenic grinding can produce smaller and more uniform particles compared to traditional grinding methods, it can help to preserve volatile compounds in the material, and can increase the throughput of the grinding process.

The process according to the invention preferably also comprise a step of separation of a liquid phase from a solid phase in order to obtain a liquid phase of a grind of a whole fresh plant and a solid phase of a grind of a whole fresh plant.

The liquid phase preferably comprises plant water and hydrophilic molecules.

The solid phase preferably comprises solid residues and lipophilic and amphiphilic molecules.

The separation step can be performed by any method well known to the person skilled in the art. Preferably, the step of separation is selected from the group consisting of filtration and centrifugation. More preferably, the step of separation is made by filtration.

Filtration is well known to the person skilled in the art and typically involves passing the mixture through a filter that retains the solid particles while allowing the liquid to pass through.

Centrifugation is well known to the person skilled in the art and typically involves spinning the mixture in a centrifuge, which generates a centrifugal force that causes the solid particles to separate from the liquid. The separated liquid can then be decanted or pipetted off from the top of the container.

The process according to the invention may further comprise a step of mixing at least one liquid phase of a grind of a whole fresh plant with at least another liquid phase of a grind of a whole fresh. The process according to the invention may further comprise a step of mixing the liquid phase of a grind of three different whole fresh plants according to the invention.

### b) Alcoholic maceration

The process according to the invention preferably further comprises a step of alcoholic maceration of a solid phase of a whole fresh plant, preferably, a solid phase of a grind of whole fresh plant according to the invention. In embodiments of the invention, the solid phase of a grind of whole fresh plant is obtained via the same grinding process as the one from which the liquid phase of a grind of a whole fresh plant is obtained. Thus, in embodiments of the invention the composition comprises at least one liquid phase of a grind of a whole fresh plant and at least one liquid phase of an alcoholic macerate of a solid phase of the grind of the same whole fresh plant.

As seen above, maceration is a process well known by the person skilled in the art which typically involves the introduction of a plant in a clean dry jar or container, the addition of a liquid, preferably cold, such as water, alcohol or oil into the jar or container in order to recover, preferably completely, the plant. The length of the maceration period can vary from few hours to several days. Once the maceration is complete, the liquid is typically filtered to obtain a liquid phase of a macerate and remove the plant residues.

Preferably, the ratio of the solid phase of a whole fresh plant according to the invention to the liquid is comprised between 1:2 (1 part of solid phase of a whole fresh plant to 2 parts of liquid) to 1: 3.

Preferably, the liquid according to the invention is a water/alcohol mixture, more preferably a 50/50 alcohol/water mixture. Alcohol can be chosen among any alcohol suitable for the maceration of a plant. Preferably, the alcohol according to the invention is selected from, methanol, ethanol, propanol, isopropanol, butanol, more preferably ethanol.

The solid phase of a whole fresh plant is preferably left in contact with the liquid for a time comprised between 5 and 25 hours, such as from 5 to 20 hours, from 5 to 15 hours, or from 5 to 10 hours.

The process according to the invention may comprise a step of mixing the liquid phase of alcoholic macerate of a solid phase of a grind of two different whole fresh plants according to the invention. The process according to the invention may comprise a step of mixing the liquid phase of alcoholic macerate of a solid phase of a grind of three different whole fresh plants according to the invention.

The process according to the invention may comprise a step a mixing at least one liquid phase of a grind of a whole fresh plant with at least one liquid phase of an alcoholic macerate of a solid phase of a grind of a whole fresh plant.

### c) Other steps

The process according to the invention may further comprise at least one step selected from the group consisting of a step of addition of at least one additive, a step of sterilization, and/or a step conditioning or packaging.

The additives according to the invention is preferably selected from the group consisting of formulation agents or additives with known, conventional use in cosmetic and dermatological compositions such as, as nonlimiting examples, emollients, colorants, film-forming agents, surfactants, perfumes, preservatives, emulsifiers, oils, glycols, vitamins such as vitamin E, UV filters, antimicrobial agents, antiseptic agents, preservative agents, antioxidant agents, and mixtures thereof. Examples of such additives include caprylic/capric triglyceride glycerin,-squalane, iron oxides, betaine, cetearyl alcohol, decyl glucoside, propanediol, citric acid, vinegar, 1,2-hexandiol, phenethyl alcohol, alpha glucan oligosaccharide, diglycerin, sodium benzoate, potassium sorbate, caprylyl glycol, cetearyl glucoside, glyceryl stearate, polyglyceryl 4 caprate stearic acid, PEG-40 stearatejojoba oil, xanthan gum, butylene glycol, vitamin C, vitamin E, beta carotènezinc oxide, panthenol, etc.

Sterilization method can be chosen among any sterilization methods well known to the person skilled in the art suitable with cosmetic ingredients. By way of example of sterilization method it is possible to cite steam sterilization, radiation sterilization, filtration sterilization, or chemical sterilization.

The type of packaging can be chosen among any suitable packaging well known to the person skilled in the art. By way of example of packaging, it is possible to cite bottles, flasks, jars, sachets or boxes.

Preferably, the process according to the invention does not include a concentration step, in particular, there is no concentration step of the plant or part of a plant according to the invention in the process according to the invention.

Advantageously, the process according to the invention makes it possible to obtain a composition comprising all the molecular diversity of the plant or the plants from which it is obtained. Thus, the composition obtained by the process according to the invention has all the biological potential of the fresh, whole plant according to the invention. In addition the conditions of culture of the plants according to the invention, i.e., the indoor culture, allow to use them in the process according to the invention preferably, within a few hours such as from 4 to 24 hours, a few days such as from 1 to 30 days of a few months, such as from 1 to 6 months after the harvest.

### Application

The composition according to the invention obtained or obtainable by the process according to the invention is particularly suited for used as or in a cosmetic composition.

The cosmetic composition according to the invention is preferably in a form suitable for a topical application, preferably on the face and on the body. Preferably, the cosmetic composition according to the invention is in the form of a cream, a serum, a mist, a gel, an oil, a milk, a balm, an emulsion, a lotion, a scrub, a mask, or a spray.

The cosmetic composition according to the invention can be used for preventing, for delaying or for combating aging of the skin and/or the appearance of signs of aging of the skin. As intended herein, the expression "signs of aging of the skin" refers to wrinkles, lines, sagging of the skin, loss of elasticity of skin fibers, withered skin, thin skin, loss of subcutaneous fat, loss of facial fat, skin that is dull and/or without radiance. The cosmetic composition according to the invention can also be used to cleanse the skin, remove impurities, reduce the appearance of blemishes, nourish the skin, moisturize the skin, make the skin more supple, make the skin firmer, reduce skin redness, improve skin elasticity, improve skin radiance, make the skin softer, make the skin more luminous, increase skin density, reduce the appearance of pores, and even out skin tone

The cosmetic composition according to the invention preferably comprises at least 80% w/w of ingredients of natural origin such as from 80 to 99% w/w, from 90 to 98% w/w, from 93 to 96% w/w of ingredients of natural origin. As intended herein, "natural origin" refers to substances or materials that occur in nature and have not been artificially created or modified.

The cosmetic composition according to the invention can be applied one or more times a day, such as from one to five times a day, for a duration of from several days to several months. Application can be localized on the zones of the body that are more particularly subject to the effects of aging, dryness or blemishes, such as the face, the breasts, the abdomen, but application can also be on the whole body. Application is preferably accompanied by massaging of the skin.

The composition according to the invention, and the composition obtained or obtainable by the process according to the invention is also particularly suited for used as a food or dietary supplement or as nutricosmetics which is also referred to as cosmeceuticals.

As intended herein, the expression "food or dietary supplement" relates to foodstuffs whose purpose is to supplement the normal diet and which constitute a concentrated source of nutrients or other substances with a nutritional or physiological effect, alone or in combination.

As intended herein, the terms "nutricosmetics" and "cosmeceuticals" refers to beauty products that are taken orally in the form of supplements, powder, pills, oil, beverages, etc., and that have the benefit of improving the appearance of external parts of the human body such as the skin, air, and nail.

Preferably, the benefits of the dietary supplement and the nutricosmetic according to the invention include improvement of skin hydration and texture, reduction on the appearance of fine lines and wrinkles, improvement of skin tone, texture and radiance, protection against degenerative skin conditions, improvement of hair and nail health, enhancement of overall health and wellness.

### Description of the Figures

Figure 1

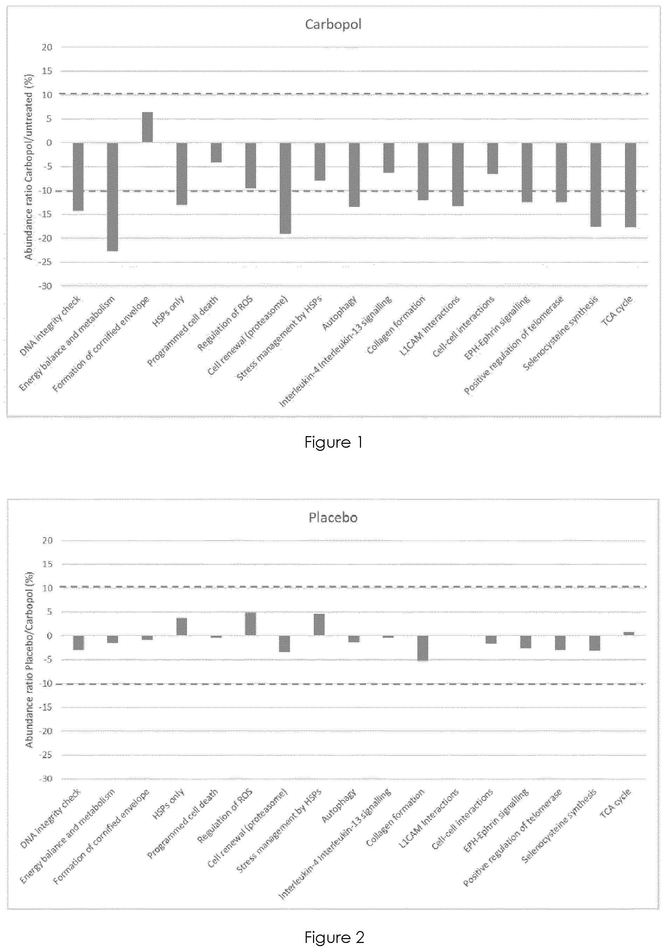

   Figure 1 shows the abundance ratio Carbopol/untreated, expressed in percentage, in function of the different pathway.
Figure 2
   Figure 1 shows the abundance ratio Placebo/Carbopol, expressed in percentage, in function of the different pathway.
Figure 3

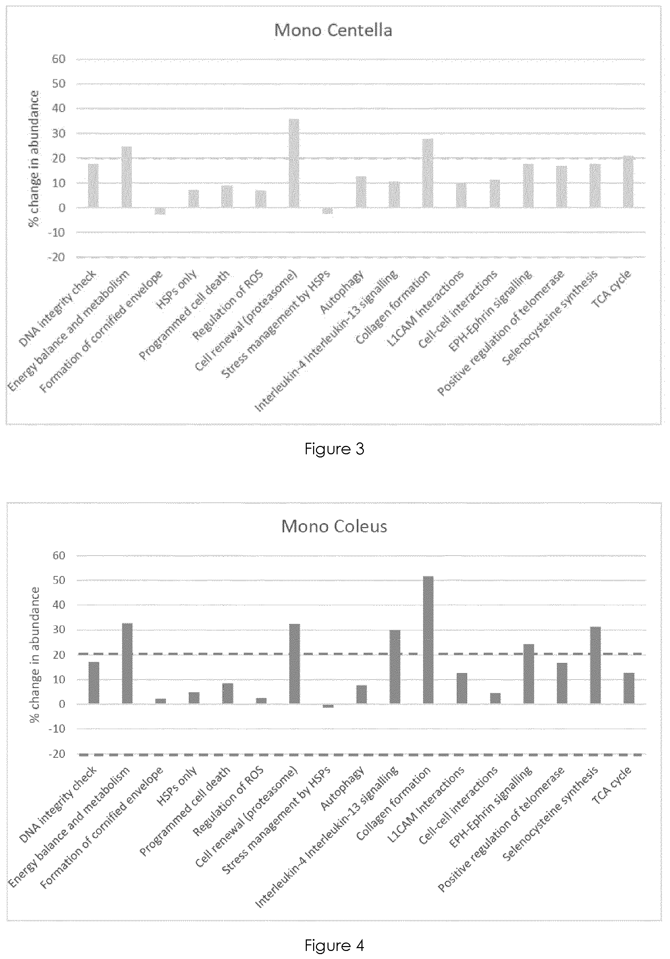

   Figure 3 shows the abundance ratio Mono Centella/untreated, expressed in percentage, in function of the different pathway.
Figure 4
   Figure 4 shows the abundance ratio Mono Coleus/untreated, expressed in percentage, in function of the different pathway.
Figure 5

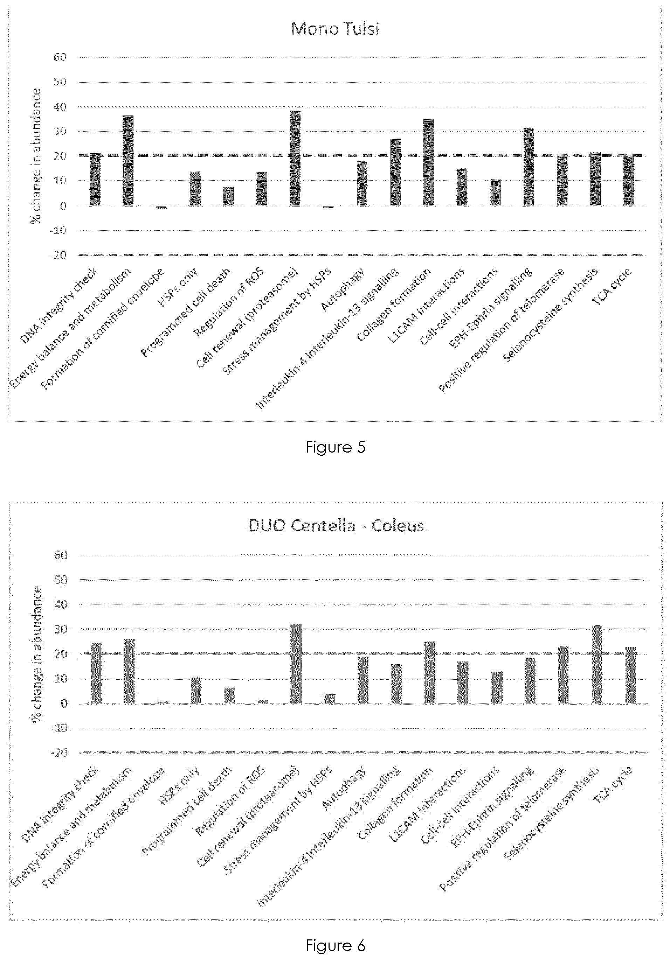

   Figure 5 shows the abundance ratio Mono Tulsi/untreated, expressed in percentage, in function of the different pathway.
Figure 6
   Figure 6 shows the abundance ratio Duo Centella-Coleus/untreated, expressed in percentage, in function of the different pathway.
Figure 7

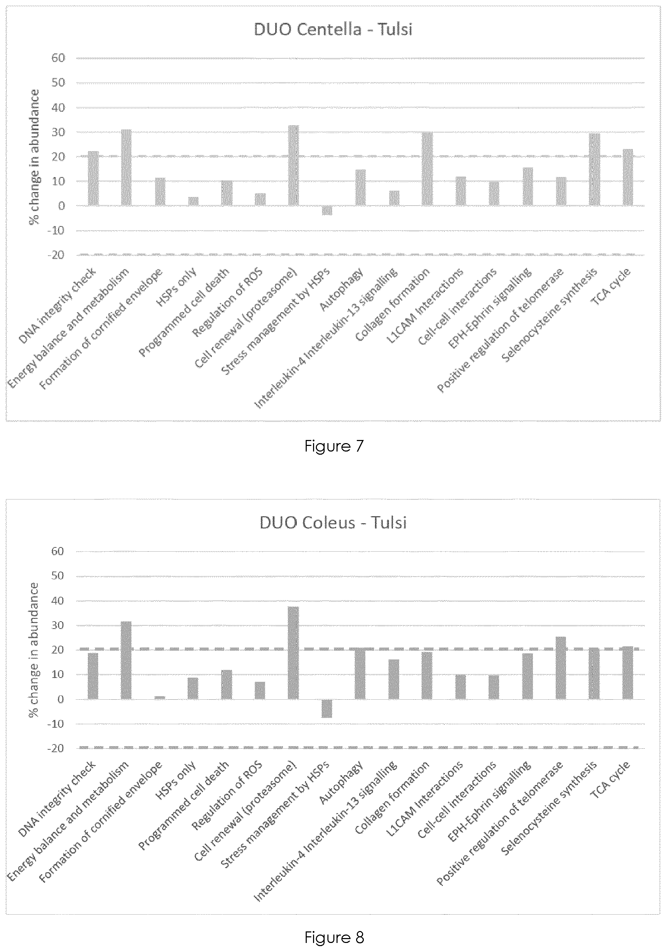

   Figure 7 shows the abundance ratio Duo Centella-Tulsi/untreated, expressed in percentage, in function of the different pathway.
Figure 8
   Figure 8 shows the abundance ratio Duo Coleus-Tulsi/untreated, expressed in percentage, in function of the different pathway.
Figure 9

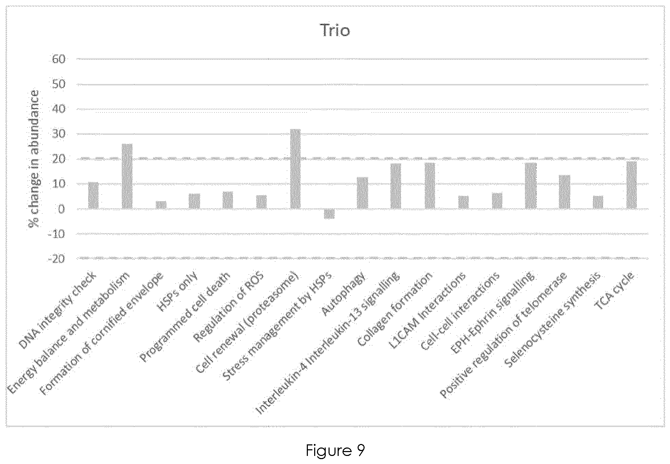

   Figure 9 shows the abundance Trio Centella-Coleus-Tusli/unrelated, expressed in percentage, in function of the different pathway.

### EXAMPLE 1

The inventors have prepared two cosmetic compositions comprising compositions according to the invention.

### 1. Preparation of a cream for topical application on the skin.

A whole fresh plant of *Coleus Forskohlii* cultured in vertical aeroponics is harvested, subjected to a cryo-grinding process and then filtered to collect the liquid phase of the grind. Phenethyl alcohol, 1,2-hexanediol, sodium benzoate, propanediol and tocopherol are added to the liquid phase of the grind of *Coleus Forskohlii.*

The same steps are performed with a whole fresh plant of *Centella asiatica* and a whole fresh plant of *Ocimum tenuiflorum.*

Then, the three liquid phases of the grind obtained from *Coleus Forskohlii, Centella* asiatica and *Ocimum tenuiflorum* are mixed together in an amount of 1/1/1. The obtained composition is referred to as Trio liquid phase of grind Centella-Coleus-Tusli. The composition obtained is then mixed with other ingredients to form a cream as disclosed in the table 1 below.

**Table 1: ingredients of the cream**

| **INCI UE** | **% Ingredient** |
|---|---|
| glycerin | 1-5% |
| xanthan gum | 0,1 - 1% |
| aqua | 19 - 55% |
| 1,2-hexanediol | 0,1 - 1% |
| panthenol | 0,1 - 1% |
| propanediol | 0,1 -5% |
| cetearyl alcohol | 1-5% |
| glyceryl stearate | |
| jojoba esters | |
| sodium stearoyl glutamate | |
| helianthus annuus seed wax | |
| aqua | |
| polyglycerin-3 | |
| tocopherol | |
| triheptanoin | 1 - 10% |
| squalane | 1 - 10% |
| pentaerythrityl tetraisostearate | 1 - 10% |
| hydroxyethyl acrylate/sodium acryloyldimethyl | 1-5% |
| taurate copolymer sorbitan isostearate | |
| Trio liquid phase of grind Centella-Coleus-Tusli. | 25 -35% |
| sodium benzoate | 0,1 - 0,5% |
| betaine | 0,1 -2% |
| alpha-glucan oligosaccharide | 0,1 - 1% |
| citric acid | 0,1 -1% |
| phenethyl alcohol | 0,1 - 1% |
| tocopherol | |
| parfum | 0,1 -0,5% |

### 2. Preparation of a serum for topical application on the skin according to the invention

A whole fresh plant of *Coleus Forskohlii* cultured in vertical aeroponics is harvested, subjected to a cryo-grinding process and then filtered to collect the liquid phase of the grind. The solid phase of the grind is separately collected. Phenethyl alcohol, 1,2-hexanediol, sodium benzoate, propanediol and tocopherol are added to the liquid phase of the grind of *Coleus Forskohlii.*

The same steps are performed with a whole fresh plant of Centella *asiatica* and a whole fresh plant of *Ocimum tenuiflorum.* Then, the three liquid phases of the grind obtained from *Coleus Forskohlii, Centella asiatica* and *Ocimum tenuiflorum* are mixed together in an amount of 1/1/1. The composition obtained is referred to as Trio liquid phase of grind Centella-Coleus-Tulsi.

The collected solid phase of *Coleus Forskohlii* is macerated in a 50/50 water/ethanol mixture, then filtered to remove plant residues. Water, alcohol, propanediol and sodium benzoate are added before proceeding to a sterilizing filtration. The obtained composition is referred to as Coleus macerate.

The same steps are reproduced with the solid phase of *Centella asiatica* and *Ocimum tenuiflorum* and the obtained compositions are respectively referred to as Centella macerate and Tulsi macerate.

The compositions are then mixed with other ingredients to form a serum as disclosed in the table 2 below.

**Table 2: ingredients of the serum**

| **INCI UE** | **% Ingredient** |
|---|---|
| Trio liquid phase of grind Centella-Coleus-Tusli | 25 - 35% |
| betaine | 0,1 -2% |
| sodium benzoate | 0,05 - 0,5% |
| polyacrylate crosspolymer-6 | 0,05-1% |
| aqua | 12,039 - 30% |
| xanthan gum | 0,15-1% |
| glycerin | 0,1 - 3% |
| propanediol | 0,05-1% |
| sodium hyaluronate | 0,1-1% |
| Coleus macerate | 3 - 25% |
| Centella macerate | 3 - 25% |
| Tulsi macerate | 3 - 25% |
| phenethyl alcohol tocopherol | 0,1 - 1% |
| parfum | 0,05 - 0,5% |
| panthenol | 0,1 - 1% |
| ethylene brassylate | 0,003750 |
| citric acid | 0,01 - 0,1% |

### EXAMPLE 2

The inventors have tested the effect of compositions according to the invention comprising at least one liquid phase of a grind of a whole fresh plant on proteome expression in human skin explants.

### A. Material and methods

### 1. Materials

The test was carried out on skin explants, a full-thickness skin biopsy embedded in a solid and nourishing matrix while its epidermal surface is left in contact with air. The skin biopsy is firmly embedded in the matrix that prevents any lateral diffusion of topically applied formulations. The donor comes from a feminine abdominoplasty, 35 years old and phototype 3.

The experiment was carried out in triplicate (n=3) in the following conditions:
- Untreated Skin explants = Untreated
- Explants + Carbopol^{®} + 2% DMSO = Carbopol
- Explants + Carbopol^{®} + 2% DMSO + placebo at 25% = Placebo
- Explants + Carbopol^{®} + 2% DMSO + Mono Centella at 25% = MCENT
- Explants + Carbopol^{®} + 2% DMSO + Mono Coleus at 25% = MCOL
- Explants + Carbopol^{®} + 2% DMSO + Mono Tulsi at 25% = MTUL
- Explants + Carbopol^{®} + 2% DMSO + Duo Centella-Coleus at 25% = DCENTCOL
- Explants + Carbopol^{®} + 2% DMSO + Duo Centella-Tulsi at 25% = DCENTUL
- Explants + Carbopol^{®} + 2% DMSO + Duo Coleus-Tulsi at 25% = DCOLTUL
- Explants + Carbopol^{®} + 2% DMSO + Trio Centella-Coleus-Tulsi at 25% = TRIO

Mono Centella, Mono Coleus, Mono Tulsi, Duo Centella-Coleus, Duo Centella-Tulsi, Duo Coleus-Tulsi and Trio Centella-Coleus-Tulsi refer to:
- compositions prepared according to the process of the invention and comprising one liquid phase of a grind of a whole fresh plant selected from *Centella asiatica, Coleus forskollii, Ocimum tenuiflorum* when it is indicated "Mono",
- two liquid phases of the grind of two different whole fresh plants selected from *Centella asiatica, Coleus forskollii, Ocimum tenuiflorum* when it is indicated "Duo", and
- three liquid phases of the grind of the three different fresh plants Centella *asiatica, Coleus forskollii, Ocimum tenuiflorum* when it is indicated "TRIO"
at a concentration of 25% w/w. Those compositions also comprise a mixture of conservators.

The placebo is a composition comprising only the mixture of conservators without the liquid phases of a grind of the whole fresh plants.

The products were applied twice a day for 5 days. After treatment, skin explants were frozen.

### 2. Method of analysis

Proteins were extracted under denatured conditions compatible with SDS-PAGE and proteomics downstream applications. Protein concentration was determined by the BCA method and standardized for all samples. The samples were separated by SDS-PAGE and digested overnight. The peptides generated were acidified and separated using an LTQ Orbitrap Fusion Lumos (Thermo Fisher), with a 146 min gradient OT/IT method.

The mass spectra were queried using Proteome Discoverer (version 2.5). The resulting MS/MS data were queried against the Homo sapiens proteome UP000005640 (20371 reviewed entries). Search parameters were as follows: monoisotopic mass; trypsin as cleavage enzyme; two max missed cleavages, carbamidomethylation of cysteine as fixed modifications; and N-terminal acetylation and oxidation of methionine as variable modifications. Results were filtered based on unique peptides >1, and global peptide scores.

A Background Based ANOVA was used to test differentially expressed proteins: this test correctly assumes that most protein abundances usually do not change in response to any one stimulus and the method automatically determines the range of protein ratios that are essentially constant between conditions and then tests each protein ratio against the median and variance derived from this background population. Analyses were performed using Proteome Discoverer 2.5 to check for overall sample quality. Protein quantitation values were exported for further analysis.

### 3. Data analysis

Proteomic analysis returned a mean total of 24420 high-quality peptides corresponding to 3247 identifiable and quantifiable skin proteins among conditions. On those, 1536 proteins were present in all samples and selected for analyses. Relevant proteins were classified by their biological processes and associated pathways using the publicly available gene ontology (GO) database provided by the Gene Ontology Consortium and Reactome. Interactome analysis and clustering was performed using a combined STRING and Cytoscape algorithm.

Proteins were clustered based on relevant biological processes and pathways and the percentage change in grouped abundance was calculated for treatments with reference to controls.

### 4. Interpretations

Interpretation is based on the premise that the tested compositions may be able to modulate specific proteins but also clusters of proteins associated with biological processes. In the analysis of the mode of action of a composition, more than one key protein may be responsible for the biological action of the composition. As such, analysis results are presented so individual proteins quantified, and included in pathways or processes' analysis, may be identified.

In skin, processes are most often accomplished by linked sets of proteins, or by a specific combination of pathways. As such, an analysis of the overall interactions and modulations of linked proteins and pathways is preferred. The association of proteins and pathways to a biological process allows to identify the processes potentially targeted by the active or formula.

### 5. Signaling pathways

During this study, several interesting pathways have emerged:
- DNA repair and cell cycle DNA checkpoints
- DNA repair and cell cycle DNA checkpoints
- Cornified envelope
- Heat Shock Proteins and stress
- Reactive oxygen species balance and regulation
- Cell renewal
- Stress management
- Interleukine 4 - Interleukine 13
- L1 CAM interaction
- Cell-cell interactions
- Selenocysteine synthesis
- TCA cycle.

### B. Results

The pathways modified by the different products are presented in the following manner:
- Effect of the Carbopol compared to the untreated;
- Effect of the Placebo compared to the Carbopol;
- Effect of the products compared to the untreated by removing the solvent effect (% change in abundance).

### 1. Carbopol Analysis

Carbopol was compared to untreated to study the difference in protein expression (see Figure 1). Carbopol decreases several pathways compared to untreated:
- DNA integrity check (-14%)
- Energy balance and metabolism (-23%)
- HSP only (-13%)
- Cell renewal (-19%)
- Autophagy (-13%)
- Collagen formation (-12%)
- L1CAM Interactions (-13%)
- EPH-Ephrin signalling (-12%)
- Positive regulation of telomerase (-12%)
- Selenocysteine synthesis (-18%)
- TCA cycle (-18%)

### 2. Placebo Analysis

Placebo was compared to Carbopol to study the difference in protein expression (see Figure 2). The placebo has no effect compared to Carbopol.

### 3. Mono Centella Analysis

The Mono Centella composition was compared to the untreated to eliminate the effect of the solvent (see Figure 3). The result shows an important increase compared to the untreated in the following pathways:
- Energy balance and metabolism (+25%)
- Cell renewal (+36%)
- Collagen formation (+28%)
- TCA cycle (+21%)

### 4. Mono Coleus Analysis

The Mono Coleus composition was compared to the untreated to eliminate the effect of the solvent (see Figure 4). The results show an effect of Mono Coleus compared to untreated on several pathways:
- Energy balance and metabolism (+33%)
- Cell renewal (+32%).
- Interleukin-4 Interleukin 13 signaling (+30%).
- Collagen formation (+52%)
- EPH-Ephrin signaling (+34%)
- Selenocysteine synthesis (+31%).

### 5. Mono Tulsi Analysis

The Mono Tulsi composition was compared to the untreated to eliminate the effect of the solvent (see Figure 5). The result shows an important increase in the following pathways:
- DNA integrity check (+21%)
- Energy balance and metabolism (+37%)
- Cell renewal (+38%).
- Interleukin-4 Interleukin 13 signaling (+27%).
- Collagen formation (+35%)
- EPH-Ephrin signaling (+31%)
- Positive regulation of telomerase (+21%)
- Selenocysteine synthesis (+22%).

### 6. Duo Centella- Coleus Analysis

The Duo Centella-Coleus composition was compared to the untreated to eliminate the effect of the solvent (see Figure 6). The result shows an important increase in the following pathways:
- DNA integrity check (+25%)
- Energy balance and metabolism (+26%)
- Cell renewal (+32%)
- Collagen formation (+25%)
- Positive regulation of telomerase (+23%)
- Selenocysteine synthesis (+31 %)
- TCA cycle (+23%)

### 7. Duo Centella- Tulsi Analysis

The Duo Centella-Tulsi composition was compared to the untreated to eliminate the effect of the solvent (see Figure 7). The result shows an important increase in the following pathways:
- DNA integrity check (+22%)
- Energy balance and metabolism (+31%)
- Cell renewal (+33%)
- Collagen formation (+30%)
- Selenocysteine synthesis (30%)
- TCA cycle (+23%)
- Formation of cornified envelope (+1 1%)

### 8. Duo Coleus- Tulsi Analysis

The Duo Coleus-Tulsi composition was compared to the untreated to eliminate the effect of the solvent (see Figure 8). The result shows an important increase in the following pathways:
- Energy balance and metabolism (+32%)
- Cell renewal (+38%)
- Autophagy (+21%)
- Positive regulation of telomerase (+25%)
- Selenocysteine synthesis (+20%)
- TCA cycle (+21%)
- Collagen formation (+19%)

### 9. Trio Centella-Coleus-Tulsi

The trio Centella-Coleus-Tulsi composition was compared to the untreated to eliminate the effect of the solvent (see Figure 9). The result shows an important increase in the following pathways:
- Energy balance and metabolism (+26%)
- Cell renewal (+32%)
- Collagen formation (+18%)

### 10. Conclusion

The aim of this study was to test the efficacy of compositions according to the invention on the skin proteome. Carbopol globally decreases protein expressions compared to the untreated. The placebo behaves like the Carbopol compared to the untreated and does not cause any additional effect. It can be seen that each composition according to the invention has an activity on several pathways. When combined together the plants have a complementary effects.

Centella compositions have an impact on energy balance and metabolism, cell renewal, collagen formation, and TCA cycle which are essential for dermis regeneration, anti-wrinkles effect and anti-aging effect.

Coleus compositions are efficient on energy balance and metabolism, cell renewal, collagen formation which are essential for dermis regeneration, anti-wrinkles effect and anti-aging effect. In addition, by acting on selenocysteine synthesis they also have a restructuring effect.

Tulsi compositions are efficient on DNA integrity check, energy balance and metabolism, cell renewal, positive regulation of telomerase, collagen formation, selenocysteine synthesis which are essential for anti-aging effect, formation of the cornified envelope.

The method of production and harvesting of the plants according to the invention makes it possible to obtain unexpected effects which were not known for these plants. For example, Coleus, which is not used in fresh form in the state of the art, has demonstrated an activity on various pathways which were not known to the skilled person for this plant.

### Example 3

The inventors have studied the potential modulation of compositions according to the invention on well-known glycobiological mechanisms.

### A. Materials and methods

### 1. Neutral monosaccharides assay (resorcinol micro-method)

The neutral sugar contents of the various products are determined according to the sulphuric resorcinol micro-method (Monsigny et al. (1988) Anal Biochem, 175: 525-530).

### 2. GLYcoPROFILE

The GLYcoPROFILE corresponds to the interaction profile of a product with a series of lectins. The analyses were carried out with a series of 20 natural and recombinant bacterial lectins and 2 human recombinant lectins (see Tables 1 and 2, below). The products to study was unlabeled so interaction profile was determined through an indirect method based on the inhibition of the interaction of specific pairs of lectins-glycans by the product to be analysed (The reference glycans used are biotinylated glycoproteins or neoglycoproteins). The inhibition profiles were determined at 3 concentrations and the analysis was repeated twice in an independent manner. Concentrations expressed on the GLYcoPROFILEs correspond to the concentrations obtained on neutral monosaccharides assay.

**Table 1: List of lectins used in GLYcoPROFILEs.**

| Short name | Common name | Glc | Man | GalNA c | Gal a | Gal b | GIcNA c | Fu c | NeuA c | Rh a |
|---|---|---|---|---|---|---|---|---|---|---|
| Con A | *Canavalia ensiformis* | + | + | | | | + | | | |
| PSA, PEA | *Pisum sativum* | + | + | | | | + | | | |
| FimH | *Escherichia coli adhesin FimH* | + | + | | | | | | | |
| BC2L-A | *Burkholderia cenocepacia lectin A* | | + | | | | | | | |
| NPA | *Narcissus pseudonarciss us Daffodil* | | + | | | | | | | |
| GNL / GNA | *Galanthus nivalis* | | + | | | | | | | |
| Lange rin | *Langerin ECD (Extra Cellular Domain)* | | + | | | | + | + | | |
| DC-SIGN | *DC-SIGN ECD (Extra Cellular Domain)* | | + | | | | | | | |
| BPA | *Bauhinia purpurea* | | | + | | | | | | |
| DBA | *Dolichos biflorus* | | | + | | | | | | |
| CJA | *Crotalaria juncea* | | | (+) | + | + | | | | |
| MPA, MPL | *Maclura pomifera* | | | + | + | + | | | | |
| AlA / Jacali n | *Artocarpus intergrifolia* | | | | + | + | | | | |
| GSL-Ib4 | *Griffonia simplicifolia isoB4* | | | | + | | | | | |
| PA-IL | *Pseudomonas aeruginosa lectin A (Lec A)* | | | | + | | | | | |
| PNA | *Arachis hypogaea* | | | | | + | | | | |
| DSL (DSA) + | *Datura stramonium* | | | | | | + | | | |
| GSL II | *Griffonia simplicifolia* | | | | | | + | | | |
| WGA | *Tritic um vulgare* | | | | | | + | | + | |
| UEA I | *Ulex europeus* | | | | | | | + | | |
| PAII-L | *Pseudomonas aeruginosa lectin B (Lec B)* | | | | | | | + | | |
| CorM | *Coregonus lavaretus marenae* | | | | | | | | | |

| | | | | | | | | | | |
|---|---|---|---|---|---|---|---|---|---|---|
| Gal: Galactose, Man: Mannose, GalNAc: Nacetylgalactosamine, GIcNAc: N-acetylglucosamine, Fuc: fucose, NeuAc: Neuraminic acid, Rha: Rhamnose, GAG: glycoaminoglycan correspond to ose abbreviations. (+) corresponds to the main inhibitors of the interaction of each lectin. | | | | | | | | | | |

**Table 2: List of recombinant human lectins with their specificity, expression and function.**

| Name | Specificity | Expression | Function |
|---|---|---|---|
| Langerin | Mannose | Langherans cells | Innate immunity |
| DC-SIGN | High mannose, fucose | Dendritic cells | Innate immunity |

### 3. Compositions tested

**Table 3: samples.**

| Sample N° | Reference |
|---|---|
| 1 | Mono Coleus (Mono Col) |
| 2 | Mono Tulsi (Mono Tul) |
| 3 | Mono Centella (Mono Cent) |
| 4 | Duo Coleus-Tulsi (Duo Col/Tul) |
| 5 | Duo Centella-Coleus (Duo Cent/Col) |
| 6 | Duo Centella-Tulsi (Duo Cent/Tul) |
| 7 | Trio Coleus-Centella-Tulsi (Trio Col/Cent/Tul) |
| 8 | Placebo Preservative |

The sample are compositions prepared according to the process of the invention and comprising one liquid phase of a grind of a whole fresh plant when it is indicated "Mono", the liquid phase of the grind of two different whole fresh plants when it is indicated "Duo" and the liquid phase of the grind of three different whole fresh plants when it is indicated "Trio", the plants being selected from *Centella asiatica, Coleus forskollii,* and *Ocimum tenuiflorum* at a concentration of 27.8% w/w.

### B. Results

### 1. Neutral monosaccharide assay (resorcinol micro-method)

The results obtained in neutral monosaccharide assays are in accordance with the composition of each sample. Indeed, the highest concentration is obtained with sample "Trio Coleus-Centella-Tulsi" with 6.2 mg/mL.

**Table 4: Neutral monosaccharides content of the sample determined by resorcinol micro-method.**

| Sample N° | Reference | Neutral monossaccharides concentration |
|---|---|---|
| 1 | Mono Coleus | 2.0 mg/ml |
| 2 | Mono Tulsi | 3.0 mg/ml |
| 3 | Mono Centella | 3.7 mg/ml |
| 4 | Duo Coleus-Tulsi | 4.2 mg/ml |
| 5 | Duo Centella-Coleus | 3.8 mg/ml |
| 6 | Duo Centella-Tulsi | 4.3 mg/ml |
| 7 | Trio Coleus-Centella-Tulsi | 6.2 mg/ml |
| 8 | Placebo Preservative | 0.6 mg/ml |

### 2. GLYcoPROFILE

Table 5 below is a summary of the main GLYcoPROFILE interaction obtained for each composition (mono/duo/trio). The interpretation was made for each GLYcoPROFILE at one neutral monosaccharide concentration (ON = 0,25 mg/mL).

**Table 5:**

| Lectins | Mono Col | Mono tul | Mono cent | Duo Col/Tu | Duo Col/Cent | Duo Cent/Tul | Trio | Placebo conservative |
|---|---|---|---|---|---|---|---|---|
| ConA | 31 ±7 | 33±6 | 23±2 | 76±6 | 61 ±6 | 39±9 | 63±11 | |
| PSA | | | | 28±3 | | 18±3 | 24±4 | |
| NPA | 43±2 | 47±14 | 44±6 | 70±22 | 49±25 | 25±21 | 36±1 | |
| GNA | 57±7 | 71±11 | 40±11 | 79±7 | 54±5 | 45±38 | 46±8 | |
| BC2LA | 50±2 | 50±2 | 49±8 | 48±9 | 80±11 | 70±7 | 66±4 | |
| FimH | 49±5 | 19±2 | 49±8 | 37±5 | 48±2 | 43±4 | 56±12 | |
| Langerin | | 38±3 | 53±2 | 52±5 | 40±5 | 51±5 | 54±16 | |
| DC-SIGN | 60±10 | 46±11 | 53±14 | 47±13 | 39±19 | 24±5 | 67±1 | |
| BPA | | | | | | | | |
| DBA | | | | | | | | |
| CJA | | | | | | | | |
| MPA | | | | | | | | |
| AlA | | | | | | | | |
| GSL-lb4 | | | 45±1 | 14±2 | 44±3 | 44±2 | 47±4 | |
| PA-IL | 15±6 | 31±3 | | 28±4 | 11±1 | 18±4 | 31±1 | |
| PNA | | | | | | | | |
| DSA | | | | | | | | |
| GSL-II | | | | | | | | |
| WGA | 30±2 | 48±7 | | 52±8 | 30±3 | 30±7 | 44±16 | |
| UEA-I | | | | | | | | |
| PA-IIL | 34±10 | 54±6 | 59±1 | 69±4 | 54±4 | 64±4 | 63±3 | |
| CorM | | | | | | | | |

In view of these results the tested compositions have potential of action notably in microbiote adhesion, immunomodulation and innate immunity, as well as permeability and would healing.
**2.1.** The composition "Mono Coleus" shows a GLYcoPROFILE with relevant lectins interactions as follow:
   - bacterial lectins FimH and BC2LA that are mannose specific and in a lesser extend with PA-IIL that are inhibited by fucose and mannose.
   - the C-type lectin DC-SIGN that is inhibited by mannose. This interaction is specific towards DC-SIGN as no interactions with the other C-type lectin, Langerin was observed.
   According to these specific interactions, this composition has potential of action on microbiote adhesion, skin immunomodulation and innate immunity by targetting specifically the dendritic cells.
**2.2.** The composition "Mono Tulsi" shows a GLYcoPROFILE with relevant lectins interactions as follow:
   - bacterial lectins BC2LA inhibited by mannose, PA-IIL and really specifically PA-IL) that is by galactose.
   - the C-type lectins DC-SIGN and Langerin that recognized mannosylated structures. But, on the contrary of Coleus extract, both lectins recognize the extract.
   - WGA, a N-acetylglucosamine (GIcNAc) specific lectin.
   This composition has a potential of action on microbiote adhesion, in skin immunomodulation and innate immunity (action on dendritic and Langerhans cells) and facilitate stratum corneum permeability and wound healing of the skin.
**2.3.** The composition "Mono Centella" shows a GLYcoPROFILE with relevant lectins interactions as follow:
   - bacterial lectins BC2LA and FimH that are both inhibited by mannose. High interaction with the PA-IIL, a lectin from P.aeruginosa that are inhibited by fucose and mannose was observed.
   - the C-type lectins DC-SIGN and Langerin that recognised mannosylated structures.
   - GSL-lb4, a lectin inhibited by galactose residue.
   This composition has potential of action on microbiote adhesion, in skin immunomodulation and innate immunity (action on dendritic and Langerhans cells) but also a potential on pigmentation modulation.
**2.4.** The GLYcoPROFILE of "Duo Colleus/Tulsi", "Duo Centella/Coleus" and "Duo Centella/Tulsi" shows high interactions that are in accordance with the sum of interactions highlighted in each extract alone.
**2.5.** The GLYcoPROFILE of "Trio Centella/Coleus/Tulsi" shows all specific interactions identified in Mono.
**2.6.** GLYcoPROFILE obtained with the product "Newskin Placebo preservative" shows no interactions on all lectins studied.

### 3. Conclusion

Each composition alone has enabled to highlight different glycan signatures. Specific interaction discovered in each single composition still exists in duo or trio. On the Trio composition which is composed of all three plants according to the invention, the interactions with the lectins are mainly higher than for each mono composition comprising only one plant, thus showing a beneficial contribution of this association.

### Example 4

The inventors have compared the phytochemical profiles of liquid phase of a grind of a whole fresh *Centella asiatica* grown according to the invention and the liquid phase of a grind of a dry extract of *Centella asiatica* grown according to conventional methods.

### A. Material and method

### 1. Samples

### 1.1. Sample according to the invention

A whole fresh plant of *Centella asiatica* cultured in vertical aeroponics is harvested, subjected to a cryo-grinding process and then filtered to collect the liquid phase of the grind. Preservatives are then added. The obtained composition is referred to as Mono-Centella

### 1.2. Sample obtained from conventional methods

The dry aerial parts of *Centella asiatica* conventionally grown in Madagascar are subjected to grinding, re-hydrated by adding nine times their weight in water, stirred and then the liquid phase is collected by filtration. Preservative are then added in the same amount as for the Mono-Centella obtained according to the invention. The obtained composition is referred to as Conventional-Centella.

### 2. Analysis

Both samples were analyzed by UHPLC coupled to a high resolution mass spectrometer (HRMS) equipped with an electrospray source (ESI), a quadrupole and a mass spectrometer (HRMS) equipped with an electrospray source (ESI), a quadrupole and a time-of-flight (Q-TOF) tube.

### Reverse phase:

- column: Luna Omega PS C18 - 1.6 µm (2.1^{∗}150mm)
- temperature: 40°C
- flow rate: 0.4 mL/min
- elution gradient:

| **Time** | 0 | 2 | 12 | 22 |
|---|---|---|---|---|
| **ACN/HCOOH 0,1 % (%)** | 0 | 0 | 100 | 100 |
| **HCOOH 0,1 % (%)** | 100 | 100 | 0 | 0 |

### HILIC mode:

- column: Nucleodur HILIC - 3.5 µm (2*150mm)
- temperature: 25°C
- flow rate: 0.2 mL/min
- elution gradient:

| **Time** | 0 | 3.75 | 5.25 | 15 | 22 | 32 |
|---|---|---|---|---|---|---|
| **ACN/HCOOH 0,1 % (%)** | 99 | 99 | 90 | 90 | 0 | 0 |
| **HCOOH 0,1 % (%)** | 1 | 1 | 0 | 0 | 100 | 100 |

### 3. Data processing

The data files obtained from the UHPLC/HRMS analysis are loaded into a calculation module that calculates the monoisotopic masses associated with each chromatographic peak; matches the data between the positive and negative modes; searches for relationships between the ions detected in the two ionization modes for each compound, and performs database queries to relate the masses to possible structures of natural compounds.

### B. Results

### 1. Results of the analysis in phase inverse

The UHPLC/HRMS chromatograms obtained for the Mono-Centella sample studied at 254 nm with the compounds detected in positive and negative modes in reverse phase show three peaks of relatively high intensity: a peak at 2.1 min, a peak at 1.5 min and a peak at 6.6 min where two compounds co-elute. One of the two compounds co-eluting at 6.58 min corresponds to the preservative benzoic acid used in the samples.

These chromatograms are compared to the UHPLC/HRMS chromatograms obtained for the Conventional-Centella studied with the compounds detected in positive and negative modes in reverse phase.

In total, 112 compounds were detected in at least one of the two samples. Of these, only 24 are common to both samples(21%). This result shows that the constituents of the plant are very different in nature. 34 compounds are only detected in Mono-Centella (30%) and 54 compounds are only detected in the Conventional-Centalla (48%).

### 2. Result of the analysis in HILIC mode

The UV chromatogram in HILIC mode of the Mono-Centella sample shows two very intense peaks eluting in the dead volume.

A analysis has been carried out by coupling the HPLC to a DEDL detector (Evaporative Light Scattering Detector). This detector allows to observe compounds that do not absorb in UV. The DEDL chromatogram of the Mono-Centella sample in HILIC mode shows the presence of several constituents. A first part of these constituents elutes between 10 and 18 minutes and the other part elutes between 21.5 and 23 minutes.

The UHPLC/HRMS chromatograms obtained for the Conventional-Centella with the peaks detected in positive and negative modes in the HILIC mode are compared with the chromatograms obtained for the Mono-Centella.

A total of 82 compounds has been detected in at least one of the two samples, of which 29 compounds are common to both samples (35%), 30 compounds are only detected in the Conventional-Centella (37%) and 23 compounds are only detected in the Mono-Centella (28%).

These results show that there is a low overlap of compounds bewteen the two samples indicating that the nature, origin and way of culturing the raw material: whole fresh plant grown in vertical farms vs dried leaves from harvesting in Madagascar have a real impact on the constituents found in the final composition.

## Claims

1. A composition comprising, or consisting of, at least one liquid phase of a grind of a whole fresh plant.

2. The composition according to claim 1, wherein the composition is a cosmetic composition.

3. The composition according to claim 1 or 2, comprising at least 1% w/w of the at least one liquid phase of a grind of a whole fresh plant.

4. The composition according to any one of claims 1 to 3, wherein the plant is grown in aeroponics.

5. The composition according to any one of claims 1 to 4, comprising the liquid phases of grinds of at least two different whole fresh plants, in particular at least three different whole fresh plants.

6. The composition according to any one of claims 1 to 5, wherein the plant is selected from the group consisting of *Centella asiatica, Coleus forskollii, Ocimum tenuiflorum* and mixtures thereof.

7. The composition according to any one of claims 1 to 6, further comprising at least one liquid phase of an alcoholic macerate of a solid phase of a whole fresh plant.

8. The composition according to claim 7, wherein the plant is selected from the group consisting of *Centella asiatica, Coleus forskollii, Ocimum tenuiflorum* and mixtures thereof.

9. The composition according to claim 7 or 8, comprising at least 3% w/w of the at least one liquid phase of an alcoholic macerate of a solid phase of a whole fresh plant.

10. The composition according to any one of claims 1 to 9,further comprising at least one additional compound selected from the group consisting of an antimicrobial agent, an antiseptic agent, a preservative agent, an antioxidant agent, and mixtures thereof.

11. A process for the preparation of a composition comprising, or consisting of, at least one liquid phase of a grind of a whole fresh plant comprising a step of cryogenic grinding of the whole fresh plant.

12. The process according to claim 11, further comprising a step of separation of a liquid phase from a solid phase.

13. The process according to claim 11 or 12, wherein the step of separation is selected from the group consisting of filtration and centrifugation.

14. The process according to any one of claims 11 to 13, further comprising a step of alcoholic maceration of the solid phase of the whole fresh plant.

15. The process according to any one of claims 11 to 14, further comprising a step of adding an additional compound selected from the group consisting of an antimicrobial agent, an antiseptic agent, a preservative agent, an antioxidant agent, and mixtures thereof.
